(19)

Europäisches Patentamt
European Patent Office
Office européen des brevets

(11) **EP 2 719 373 B1**

(12) **EUROPEAN PATENT SPECIFICATION**

(45) Date of publication and mention
of the grant of the patent:
**19.04.2017 Bulletin 2017/16**

(51) Int Cl.:
***A61K 9/00*** *(2006.01)*  ***A61K 9/20*** *(2006.01)*

(21) Application number: **12188344.1**

(22) Date of filing: **12.10.2012**

(54) **Fast disintegrating solid dosage form formulation comprising functionalized calcium carbonate and method of their manufacture**

Sich schnell auflösende Feststoffdosierungsformformulierung mit funktionalisiertem Calciumcarbonat und Herstellungsverfahren dafür

Forme posologique solide à désintégration rapide comprenant une formulation de carbonate de calcium fonctionnalisée et leur procédé de fabrication

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(43) Date of publication of application:
**16.04.2014 Bulletin 2014/16**

(73) Proprietor: **Omya International AG**
**4665 Oftringen (CH)**

(72) Inventors:
• **Gerard, Daniel E.**
**4058 Basel (CH)**
• **Schölkopf, Joachim**
**5727 Oberkulm (CH)**

• **Gane, Patrick A.C.**
**4852 Rothrist (CH)**
• **Stirnimann, Tanja**
**4800 Zofingen (CH)**
• **Alles, Rainer**
**4058 Basel (CH)**
• **Puchkov, Maxim**
**4148 Pfeffingen (CH)**
• **Huwyler, Jörg**
**4144 Arlesheim (CH)**

(74) Representative: **Glas, Holger**
**Maiwald Patentanwalts GmbH**
**Elisenhof**
**Elisenstrasse 3**
**80335 München (DE)**

(56) References cited:
**WO-A1-2010/037753      US-A1- 2005 244 493**

**Description**

Field of the invention

The present invention relates to fast disintegrating solid dosage form formulation and to the method of their manufacture. The fast disintegrating solid dosage form formulation may deliver an active ingredient or inactive precursor in an easy way in form of tablets in the oral cavity by fast disintegration in an aqueous environment. According to The International Pharmacopoeia: "disintegration is defined as the state in which no residue of the tablet or capsule, except fragments of undissolved coating or capsule shell, remains on the screen of the test apparatus or, if any other residue remains, it consists of a soft mass having no palpably firm, unmoistened core". [Source: The International Pharmacopoeia, WHO, 2011]

Background of the Invention

There are various types of oral administrative medicines. Such medicine encompasses tablets, capsules, granules, powder, syrups, and gels, among others. However, such oral administrative medicines may cause problems as mentioned on the following. Geriatric, pediatric and patients with some sort of disabilities of swallowing, e.g. weak swallowing power, have problems with the intake of tablets and capsules. As to granules and powders, they may cause an unpleasant feeling in the mouth or may, when intake is uncoordinated, erroneously be inhaled and end up in the respiratory tract or lungs, causing irritations, indispositions or even pain. Furthermore, they cannot be taken in the absence of a liquid such as water, because such a liquid is required for dosing. Syrups and gels are difficult to dose without any helping means, e.g. spoons or syringes, and thus make it difficult for older people or children to measure the correct dosing.

Therefore, the demand for orally rapid or fast disintegrating dosage forms such as tablets, mini-tablets, granules or pellets has increased within the past years. Orally rapid or fast disintegrating tablets, when placed in the mouth and dispersing rapidly in saliva without the need of liquid and which can be readily swallowed, provide for a simple form of self-administration and dosing. For such tablets the European Pharmacopoeia (01/2008:1154) adopted the term orodispersible tablets with the following definition: "Orodispersible tablets are uncoated tablets intended to be placed in the mouth where they disperse rapidly before being swallowed".

Disintegration shall take place within 3 minutes. The Food and Drug Administration (FDA) of the United States require an *in vitro* disintegration time of approximately of 30 seconds or less. This dosage form is therefore not only suitable for geriatric or pediatric patients, but also for mentally ill, bedridden, developmentally disabled patients or patients with underlying diseases which disrupts swallowing ability or patients with persistent nausea and vomiting, and patients who are travelling and thus do not have easy access to water.

For fast dissolving or disintegrating tablets the European Pharmacopoeia (01/2008:1154) adopted the term dispersible tablets with the following definition: "Dispersible tablets are uncoated or film-coated tablets intended to be dispersed in water before administration, giving a homogeneous dispersion". Dispersible tables disintegrate within 3 min., using water at 15-25°C.

Such orodispersible tablets, also known as (ODTs), or fast dispersible tablets known as (FDTs) can be prepared by different techniques, such as freeze drying, molding, spray drying, mass extrusion or compressing. Depending on the technique and composition, the final forms have various dissolution properties, such as rapid dissolution while at the same time having low mechanical strength, i.e. low hardness, and therefore a high friability. Further disadvantages are high costs of production, low drug content and possibly also limitations in stability.

A further property of the ODTs or FDTs should be, that they have a sufficient hardness and/or friability. Hardness is required, in order to push a tablet form a PTP package (Press Through Package), and a good friability is required when tablets are bottled or transported in containers to minimize abrasion. Further to this, hardness is required if tablets should be film coated. This is often a great need as this affects stability and as a consequence also the shelf life of a product.

There are currently several fast-dissolving products on the market. US 4,134,943, US 5,595,761, US 5,635,210, US 5,807,576, and US 6,066,337 refer to rapidly dissolving tables, dosing forms and methods for producing them. The particulate support matrix was made from hydrolyzed and non-hydrolyzed gelatin. Before forming the particulate support matrix into the tablet, a drug, medication, or pharmaceutical, and any desired flavoring agent is added. Optionally, an effervescent material is added to assist in the initial stage of disintegration of the particles of the tablet. The tablets may further comprise one or more excipients which can be chosen from those known in the art, including flavors, diluents, colors, binders, fillers, compaction vehicles, effervescent agents, and non-effervescent disintegrants. The tablets may be formed by direct compression.

WO 2010/037753 of the same applicant refers to surface modified calcium carbonate as new controlled release active agent carrier. The surface modified calcium carbonate was made into tablets, tooth paste and bath bombs or bath tablets. However said tablets, bath bombs or bath tablets, did not dissolve rapidly, rather the opposite, the needed several minutes to dissolve.

The above mentioned problems have now been solved by the present invention.

## Summary of the invention

The present invention relates to fast disintegrating dosage form in the form of a tablet comprising functionalized natural and/or synthetic calcium carbonate (FCC) as novel pharmaceutical excipient. Such intrabuccally fast disintegrating tablets are also known as orally dispersible or disintegrating tablets (ODTs) or as orally fast dissolving tablets (FDTs). Such ODTs or FDTs are solid single-unit dosage forms, which instantaneous disperse or dissolve in an aqueous environment such as the saliva. However, the fast dissolving dosage forms of the present invention are not limited to intrabuccal or oral administration only. The fast disintegrating dosage forms can be also dissolved in another aqueous environment such as tap water, tea or juices. The fast dissolving dosage forms comprising functionalized natural and/or synthetic calcium carbonate (FCC) are therefore useful in pharmaceutical and confectionary fields. The functionalized natural or synthetic calcium carbonate (FCC) can be prepared from either natural ground calcium carbonate comprising mineral or from synthetic calcium carbonate, sometimes also named as precipitated calcium carbonate, or mixtures thereof.

The present invention also comprises a method for the preparation of the fast disintegrating dosage form in the form of a tablet by direct compression, extrusion, granulation or roller compaction.

The present invention relates also to the use of FCC in fast disintegrating dosage form.

## Description of the Invention

The present invention relates to intrabuccally fast disintegrating and disintegrating dosage forms in the form of a tablet comprising functionalized natural and/or synthetic calcium carbonate (FCC) as novel pharmaceutical excipient. The fast disintegrating dosage forms of the present inventions comprises functionalized natural or synthetic calcium carbonate or functionalized blend of natural and synthetic calcium carbonates, at least one active ingredient and at least one disintegrant, wherein said functionalized natural or synthetic calcium carbonate is a reaction product of natural or synthetic calcium carbonate or mixtures thereof with carbon dioxide and one or more acids, wherein the carbon dioxide is formed in situ by the acid treatment and/or is supplied from an external source, wherein the at least one disintegrant is selected from the group comprising modified cellulose gums, insoluble cross-linked polyvinylpyrrolidones, starch glycolates or mixtures thereof and wherein the tablet has a hardness in the range from 40 to 100 N and a corresponding tensile strength in the range of 0.4 to 1.3 MPa and the tablet disintegrates in less than or in 3 minutes, preferably in less than or in 2 minutes, more preferably in less than or in 1 minute, still more preferably in less than or in 30 seconds, when introduced into an aqueous environment. Disintegration times may even do down to 20 seconds or less, such as a disintegration time between and including 10 to 20 seconds.

The source of natural calcium carbonate for preparing the functionalized calcium carbonate (FCC) is selected from the group of marble, calcite, chalk, limestone and dolomite and/or mixtures thereof.

In a particular embodiment the synthetic calcium carbonate for preparing the functionalized calcium carbonate is precipitated calcium carbonate (PCC) comprising aragonitic, vateritic and/or calcitic mineralogical crystals forms, especially prismatic, rhombohedral or scalenohedral PCC or mixtures thereof.

The process for preparing the functionalized natural and/or synthetic calcium carbonate (FCC) will now be further described.

In a preferred embodiment, the natural or synthetic calcium carbonate is ground prior to the treatment with one or more acids and carbon dioxide. The grinding step can be carried out with any conventional grinding device such as grinding mill known to the skilled person.

In a preferred process, the natural or synthetic calcium carbonate, either finely divided, such as grinding, or not, is suspended in water. Preferably the slurry has a content of natural or synthetic calcium carbonate within the range of 1 wt-% to 80 wt-%, more preferably 3 wt-% to 60 wt-%, and still more preferably from 5 wt-% to 40 wt-%, based on the weight of the slurry.

In a next step, an acid is added to the aqueous suspension containing the natural or synthetic calcium carbonate. Preferably, the acid has a $pK_a$ at 25°C of 2.5 or less. If the $pK_a$ at 25°C is 0 or less, the acid is preferably selected from sulphuric acid, hydrochloric acid, or mixtures thereof. If the $pK_a$ at 25°C is from 0 to 2.5, the acid or its metal salt is preferably selected from $H_2SO_3$, $HSO_4^-M^+$, $H_3PO_4$, $H_2PO_4^-M^+$ or mixtures thereof, wherein $M^+$ can be $Na^+$ and/or $K^+$.

In another embodiment, the acid is preferably phosphoric acid in combination with acetic, formic or citric acid or acid salts thereof.

More preferably, the acid is phosphoric acid alone.

The one or more acids can be added to the suspension as a concentrated solution or a more diluted solution. Preferably, the molar ratio of $H_3O^+$ ion to the natural or synthetic calcium carbonate is from 0.1 to 2.

As an alternative, it is also possible to add the acid to the water before the natural or synthetic calcium carbonate is suspended.

In a next step, the natural or synthetic calcium carbonate is treated with carbon dioxide. If a strong acid such as sulphuric acid or hydrochloric acid or a medium-strong acid is used for the acid treatment of the natural or synthetic calcium carbonate, the carbon dioxide is automatically formed. Alternatively or additionally, the carbon dioxide can be supplied from an external source.

Acid treatment and treatment with carbon dioxide can be carried out simultaneously which is the case when a strong acid is used. It is also possible to carry out acid treatment first, e.g. with a medium strong acid having a $pK_a$ in the range of 0 to 2.5, followed by treatment with carbon dioxide supplied from an external source.

Preferably, the concentration of gaseous carbon dioxide in the suspension is, in terms of volume, such that the ratio (volume of suspension):(volume of gaseous $CO_2$) is from 1:0.05 to 1:20, even more preferably 1:0.05 to 1:5.

In a preferred embodiment, the acid treatment step and/or the carbon dioxide treatment step are repeated at least once, more preferably several times.

Subsequent to the acid treatment and carbon dioxide treatment, the pH of the aqueous suspension, measured at 20°C, naturally reaches a value of greater than 6.0, preferably greater than 6.5, more preferably greater than 7.0, even more preferably greater than 7.5, thereby preparing the functionalized natural or synthetic calcium carbonate as an aqueous suspension having a pH of greater than 6.0, preferably greater than 6.5, more preferably greater than 7.0, even more preferably greater than 7.5. If the aqueous suspension is allowed to reach equilibrium, the pH is greater than 7. A pH of greater than 6.0 can be adjusted without the addition of a base when stirring of the aqueous suspension is continued for a sufficient time period, preferably 1 hour to 10 hours, more preferably 1 to 5 hours.

Alternatively, prior to reaching equilibrium, which occurs at a pH greater than 7, the pH of the aqueous suspension may be increased to a value greater than 6 by adding a base subsequent to carbon dioxide treatment. Any conventional base such as sodium hydroxide or potassium hydroxide can be used.

Further details about the preparation of the functionalized natural calcium carbonate are disclosed in WO 00/39222 and US 2004/0020410 A1, wherein the functionalized natural calcium carbonate is described as a filler for paper manufacture, the content of these references herewith being included in the present application.

Yet a different process for the preparation of functionalized natural calcium carbonate suitable for the present invention is disclosed in EP 2 264 108 of the same applicant, the content of this reference being herewith included in the present application. Basically, the process for preparing a functionalized calcium carbonate in an aqueous environment comprises the following step:

a) providing at least one ground natural calcium carbonate (GNCC);
b) providing at least one water-soluble acid;
c) providing gaseous $CO_2$;
d) contacting said GNCC of step a) with said acid of step b) and with said $CO_2$ of step c);

characterized in that:

(i) said acid (s) of step b) each having a pKa of greater than 2.5 and less than or equal to 7, when measured at 20°C, associated with the ionisation of their first available hydrogen, and a corresponding anion formed on loss of this first available hydrogen capable of forming water-soluble calcium salts;
(ii) following contacting said acids(s) with said GNCC, at least one water-soluble salt, which in the case of a hydrogen-containing salt has a pKa of greater than 7, when measured at 20°C, associated with the ionisation of the first available hydrogen, and the salt anion of which is capable of forming water-insoluble calcium salts, is additionally provided.

The ground natural calcium carbonate is selected form the group consisting of marble, chalk, calcite, limestone and mixtures thereof. Suitable particle sizes of the GNCC can be easily found in the cited reference, as well as the water-soluble acids, e.g. particles with weight median diameter of 0.01 to 10μm, and acids selected from acetic acids, formic acid, propanoic acid, and mixtures thereof.

Similarly, functionalized precipitated calcium carbonate is obtained. As can be taken in detail from EP 2 070 991 B1 from the same applicant, wherein functionalized precipitated calcium carbonate is obtained by contacting precipitated calcium carbonate with $H_3O^+$ ions and with anions being solubilized in an aqueous medium and being capable of forming water-insoluble calcium salts, in an aqueous medium to form a slurry of functionalized precipitated calcium carbonate, wherein said functionalized precipitated calcium carbonate comprises an insoluble, at least partially crystalline calcium salt of said anion formed on the surface of at least part of the precipitated calcium carbonate.

Said solubilized calcium ions correspond to an excess of solubilized calcium ions relative to the solubilized calcium ions naturally generated on dissolution of precipitated calcium carbonate by $H_3O^+$ ions, where said $H_3O^+$ ions

are provided solely in the form of a counter ion to the anion, i.e. via the addition of the anion in the form of an acid or non-calcium acid salt, and in absence of any further calcium ion or calcium ion generating source.

Said excess solubilized calcium ions are preferably provided by the addition of a soluble neutral or acid calcium salt, or by the addition of an acid or a neutral or acid non-calcium salt which generates a soluble neutral or acid calcium salt in situ.

Said $H_3O^+$ ions may be provided by the addition of an acid or an acid salt of said anion, or the addition of an acid or an acid salt which simultaneously serves to provide all or part of said excess solubilized calcium ions.

In a preferred embodiment of the preparation of the functionalized natural or synthetic calcium carbonate, the natural or synthetic calcium carbonate is reacted with the acid and/or the carbon dioxide in the presence of at least one compound selected from the group consisting of aluminium sulfates, silicate, silica, aluminium hydroxide, earth alkali aluminate such as sodium or potassium aluminate, magnesium oxide, or mixtures thereof. Preferably, the at least one silicate is selected from an aluminium silicate, a calcium silicate, or an earth alkali metal silicate. These components can be added to an aqueous suspension comprising the natural or synthetic calcium carbonate before adding the acid and/or carbon dioxide.

Alternatively, the silicate and/or silica and/or aluminium hydroxide and/or earth alkali aluminate and/or magnesium oxide component(s) can be added to the aqueous suspension of natural or synthetic calcium carbonate while the reaction of natural or synthetic calcium carbonate with an acid and carbon dioxide has already started. Further details about the preparation of the functionalized natural or synthetic calcium carbonate in the presence of at least one silicate and/or silica and/or aluminium hydroxide and/or earth alkali aluminate component(s) are disclosed in WO 2004/083316, the content of this reference herewith being included in the present application.

The functionalized natural or synthetic calcium carbonate can be kept in suspension, optionally further stabilised by a dispersant. Conventional dispersants known to the skilled person can be used. A preferred dispersant is polyacrylic acid or partially or totally neutralized polyacrylic acid.

Alternatively, the aqueous suspension described above can be dried, thereby obtaining the solid (i.e. dry or containing as little water that it is not in a fluid form) functionalized natural or synthetic calcium carbonate in the form of granules or a powder.

In a preferred embodiment, the functionalized natural or synthetic calcium carbonate has a BET specific surface area of from 5 $m^2$/g to 200 $m^2$/g, preferably 15 $m^2$/g to 150 $m^2$/g, more preferably 40 $m^2$/g to 100 $m^2$/g, measured using nitrogen and the BET method according to ISO 9277:2010.

Furthermore, it is preferred that the functionalized natural or synthetic calcium carbonate has a weight median grain diameter of from 0.1 to 50 $\mu$m, preferably from 0.5 to 25 $\mu$m, more preferably from 0.8 to 20 $\mu$m, still more preferably from 1 to 15 $\mu$m, measured using Malvern Mastersizer X long bed.

In a preferred embodiment, the functionalized natural or synthetic calcium carbonate (FCC) has a BET specific surface area within the range of 5 $m^2$/g to 200 $m^2$/g and a weight median grain diameter within the range of 0.1 $\mu$m to 50 $\mu$m. More preferably, the specific surface area is within the range of 15 $m^2$/g to 75 $m^2$/g and the weight median grain diameter is within the range of 0.5 $\mu$m to 25 $\mu$m. Even more preferably, the specific surface area is within the range of 25 $m^2$/g to 55 $m^2$/g and the weight median grain diameter is within the range of 1 $\mu$m to 15 $\mu$m

By the above described process natural or synthetic calcium carbonate is modified to enhance on one hand the porosity of the FCC and on the other hand to enlarge the surface area. The FCC absorbs water at a faster rate compared to conventional calcium carbonate and is able to absorb ten times more fluid than conventional calcium carbonate. Reference is made to C.J.Ridgway et al. "Modified calcium carbonate coatings with rapid absorption and extensive liquid uptake capacity", Colloids and Surfaces A: Physicochemical and Engineering Aspects, vol. 236, no. 1-3, pp. 91-102, Apr. 2004.

In this respect, it is believed that because of the intra and interpore structure of the functionalized calcium carbonate, this material is a superior agent to transport liquids through the pores faster over time relative non-functionalized calcium carbonate.

Thus, the absorption and release characteristics can be controlled by the pore size and/or pore volume and/or surface area.

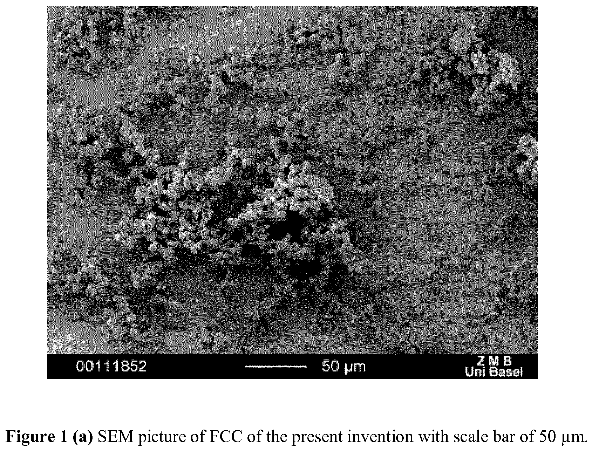

**Figure 1 (a)** SEM picture of FCC of the present invention with scale bar of 50 $\mu$m.

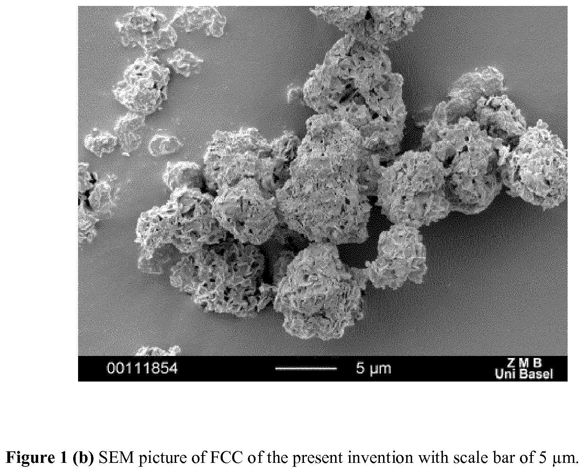

**Figure 1 (b)** SEM picture of FCC of the present invention with scale bar of 50 $\mu$m.

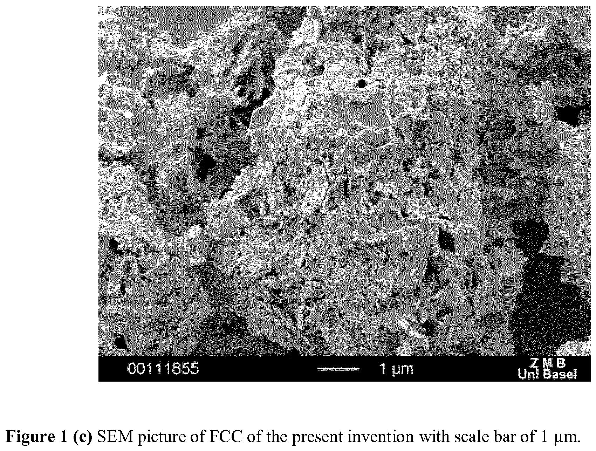

**Figure 1 (c)** SEM picture of FCC of the present invention with scale bar of 50 $\mu$m.

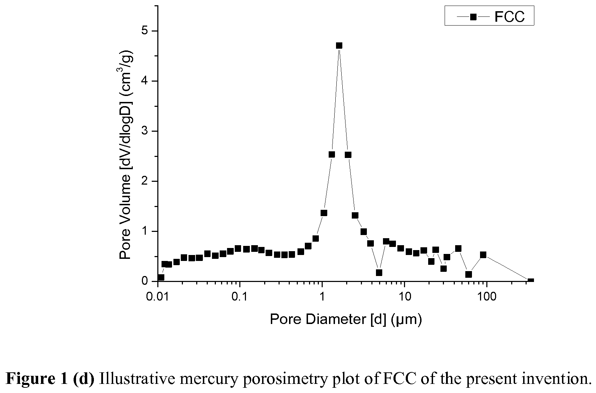

**Figure 1 (d)** Illustrative mercury porosimetry plot of FCC of the present invention.

Figure 1 (a-c) shows SEM pictures of FCC with different magnifications. The size of the FCC particles was around 7 $\mu$m. The particles showed a multitude of thin lamellae that formed a porous meshwork.

Preferably, the functionalized natural or synthetic calcium carbonate has an intra-particle porosity within the range from 20 vol.-% to 99 vol.-%, preferably from 30 vol.-% to 70 vol.-%, more preferably from 40 vol.-% to 60 vol.-%

calculated from a mercury porosimetry measurement. From the bimodal derivative pore size distribution curve the lowest point between the peaks indicates the diameter where the intra and inter-particle pore volumes can be separated. The pore volume at diameters greater than this diameter is the pore volume associated with the inter-particle pores. The total pore volume minus this inter particle pore volume gives the intra particle pore volume from which the intra particle porosity can be calculated, preferably as a fraction of the solid material volume, as described in Transport in Porous Media (2006) 63: 239-259.

Thus, the intra-particle porosity determined as the pore volume per unit particle volume is within the range of from 20 vol.-% to 99 vol.-%, preferably from 30 vol.-% to 80 vol.-%, more preferably from 40 vol.-% to 70 vol.-%, most preferably from 50 vol.% to 65 vol.%.

As already mentioned absorption and release of liquids is essentially controlled by the pore size, wherein the internal pore size is defined as a distribution of pore sizes ranging from as low as 0.01 to 1 $\mu$m. Internal pore size has to be understood as the pores present on individual particles, compared to intra pore size, meaning the voids between individual particles.

In order to promote rapid disintegration of fast disintegrating dosage forms a disintegrating agent or disintegrants are commonly used. Such disintegrants are known to the skilled person as well as their mechanisms of action.

There are three major mechanisms and factors affecting tablet disintegration:

- Swelling
- Porosity and Capillary Action
- Deformation

Swelling

Although not all effective disintegrants swell in contact with water, swelling is believed to be a mechanism in which certain disintegrating agents (such as starch) impart a disintegrating effect. By swelling in contact with water, the adhesiveness of other ingredients in a tablet is overcome causing the tablet to fall apart.

Porosity & Capillary Action

Effective disintegrants that do not swell are believed to impart their disintegrating action through porosity and capillary action. Tablet porosity provides pathways for the penetration of fluid into tablets. The disintegrant particles, sometimes with low cohesiveness and compressibility, themselves act to enhance porosity and provide these pathways into the tablet. Liquid is drawn up into these pathways through capillary action and rupture the interparticulate bonds causing the tablet to break apart.

Deformation

Starch grains are generally thought to be elastic in nature, meaning that grains that are deformed under pressure will return to their original shape when that pressure is removed. But, with the compression forces involved in tableting, these grains are believed to be deformed more permanently and are said to be rich in energy with this energy being released upon exposure to water. In other words, the ability for starch to swell is higher in rich energy starch grains than it is for starch grains that have not been deformed under pressure.

It is believed that no single mechanism is responsible for the action of most disintegrants. But rather, it is most likely the result of inter-relationships between these major mechanisms.

Within the context of the present invention the term disintegrant or disintegrating agent encompass disintegrants exhibiting the above mentioned mechanisms.

The fast disintegrating dosage forms according to the present invention comprises at least one disintegrant exhibiting one of the mechanisms described above. The fast disintegrating dosage forms according to the present invention comprises at least one disintegrant selected form the group comprising modified cellulose gums, insoluble cross-linked polyvinylpyrrolidones, starch glycolates or mixtures thereof.

Examples of disintegrants are: Ac-Di-Sol®, FMC, USA - which is a modified cellulose gum; Kollidon®CL, BASF, Germany - which is an insoluble crosslinked polyvinlypyrrolidone; Vivastar®, JRS, Germany - which is a sodium starch glycolate; MCC Polymorph II (MCC SANAQ Burst®) - Pharmatrans Sanaq AG, Switzerland - which is a stable crystal polymorph type II of Microcrystalline cellulose, MCC SANAQ 102 as standard microcrystalline cellulose (MCC).

It lies within the understanding of the skilled person that the mentioned disintegrants are of mere illustrative character and are not intended to be of limiting character.

The at least one disintegrant is present in the range from about 0.3 wt% to about 10 wt%, preferably from about 0.5 wt% to about 8 wt%, more preferably from about 1 wt% to about 5 wt% based on the weight of functionalized natural or synthetic calcium carbonate. In particular embodiment, the disintegrant is present in an amount of 3 wt% to 4 wt% based on the weight of functionalized natural or synthetic calcium carbonate.

The fast disintegrating dosage forms of the present invention may further comprise, but is not limited to, additional compounds such as fillers, binders, diluents, adhesives, lubricants or miscellaneous materials such as buffers and adsorbents.

Within the context of the present invention, an active ingredient encompasses also inactive pharmaceutical and biological precursors which will be activated at a later stage.

The fast disintegrating dosage forms of the present invention may still further comprise at least one active ingredient selected from the group comprising pharmaceutically active ingredients, inactive pharmaceutical precursors, biologically active ingredients, inactive biological precursors or combinations thereof.

The activation of such inactive precursors is known to the skilled person and commonly in use, e.g. activation in the stomach and/or gestro-interstinal pathway-such as acidic activation, tryptic-, chimotryptic or pepsinogenic cleavage.

It lies within the understanding of the skilled person that the mentioned activation methods are of mere illustrative character and are not intended to be of limiting character.

The fast disintegrating dosage forms of the present invention may further comprise natural or synthetic scenting agents, natural or synthetic flavoring agents, natural or synthetic coloring agents, natural or synthetic sweeteners and/or mixtures thereof.

Suitable natural or synthetic scenting agents include one or more volatilized chemical compounds, generally at a very low concentration, that humans or other animals perceive by the sense of olfaction.

Suitable natural or synthetic flavoring agents include but are not limited to mints, such as peppermint, menthol, vanilla, cinnamon, various fruit flavors, both individual or mixed, essential oils such as thymol, eucalyptol, menthol, and methyl salicylate, allylpyrazine, methoxypyrazines, 2-isobutyl-3 methoxypyrazine, acetyl-L-pyrazines, 2-acetoxy pyrazine, aldehydes, alcohols , esters, ketones, pyrazines, phenolics, terpenoids and mixtures thereof.

The flavoring agents are generally utilized in amounts that will vary depending upon the individual flavor, and may, for example, range in amount of about 0.5% to about 4% by weight of the final composition.

Suitable natural or synthetic coloring agents include, but are not limited to, titanium dioxide, flavone dyes, isoquinoline dyes, polyene colorants, pyran colorants, naphthochinone dyes, chinone and anthrachinone dyes, chromene dyes, benzophyrone dyes as well as indigoid dyes and indole colorants. Examples thereof are caramel coloring , annatto, chlorophyllin, cochineal, betanin, turmeric, saffron, paprika, lycopene, pandan and butterfly pea.

Suitable natural or synthetic sweeteners include but are not limited to xylose, ribose, glucose, mannose, galactose, fructose, dextrose, sucrose, sugar, maltose, partially hydrolyzed starch, or corn syrup solid, and sugar alcohols such as sorbitol, xylitol, mannitol, and mixtures thereof; water soluble artificial sweeteners such as the soluble saccharin salts, i.e. sodium, or calcium saccharin salts, cyclamate salts, acesulfam-K and the like, and the free acid form of saccharin and aspartame based sweeteners such as L-aspartyl-phenylalanine methyl ester, Alitame® or Neotame®.

In general, the amount of sweetener will vary with the desired amount of sweeteners selected for a particular tablet composition.

To further promote rapid dissolution of the fast disintegrating dosage forms of the present invention, said dosage form may further comprise at least one effervescing agent. Said effervescing agent can be selected from the group comprising acids, acid salts, or hydrogen carbonates.

Sodium Bicarbonate in combination with citric or tartaric acids is used as an "effervescent" disintegrant.

The present invention is further related to the use of functionalized calcium carbonate (FCC) in fast disintegrating dosage forms. Particularly to the use in orally fast dispersible/disintegrating dosage forms or fast dispersible dosage forms for dissolution in tap water, tea or juices. Said fast disintegrating dosage forms comprising tablets.

In a preferred embodiment fast disintegrating dosage form is in form of a tablet. Said tablet being made by direct compression. High shear and fluidized bed granulation process as well as roller compaction are suitable processing methods as well.

The tablet of the present invention made by direct compression has a hardness in the range of 40 to 100N and a tensile strength in the range of 0.4 to 1.3 MPa.

The tensile strength σ (MPa) is calculated in accordance to the following equation:

$$\sigma_t = \frac{2 \cdot F}{\pi \cdot d \cdot h}$$

wherein F is the measured tablet hardness (N), d is the tablet diameter, and h is the tablet height.

The present invention is now further described by way of examples.

<u>Examples</u>

*True density and median particles diameters*

Density and median grain particle diameter were determined for the fillers (F) and disintegrants (D) used in the present invention. Table 1 provides for the density and median grain diameter.
Within the context of the present invention, true density means the density as determined by helium pycnometric measurements.

Table 1. True density and median grain particle diameter

| Substance | Use | True density (g/cm$^3$) | Median particle diameter ($\mu$m) $\pm$ SD |
|---|---|---|---|
| FCC | F | 2.7382 | 7.28 $\pm$ 0.05 |
| Barcroft CS90 | F | 2.5233 | 163.52 $\pm$ 10.00 |
| MCC | F | 1.5583 | 120.65 $\pm$ 1.31 |
| FlowLac | F | 1.5412 | 150.37 $\pm$ 2.19 |
| UICEL | F + D | 1.5337 | 64.04 $\pm$ 0.14 |
| AcDiSol | D | 1.5996 | 43.74 $\pm$ 0.06 |
| VivaStar | D | 1.4778 | 41.20 $\pm$ 0.12 |
| Kollidon CL | D | 1.2374 | 91.64 $\pm$ 0.81 |

Table 1 shows the true densities and the medians of the particle diameter of the used substances. With the BET method a specific surface area of 62.14 $\pm$ 0.19 m$^2$/g was measured for the FCC particles.

*Tablet preparation*

All powders and formulations were mixed by using a tumbling mixer (Turbula T2C, Switzerland) for 10 min at 32 rpm. The tablets were compressed by a single punch press (Korsch EK0, Berlin) with 11 mm round flat tooling. The punch gap was adjusted to compact 500 mg of FCC powder into a tablet with hardness of 100 N. The resulting tablet had a height of 5.30 mm. This setting for the punch gap was kept constant for all the other mixtures. The target hardness of 100 N was obtained by changing the mass of the compacts. The tablets were kept at constant temperature and humidity in closed containers to allow enough time for expansion. Table 2 provides for the tablet formulations and tablet properties.

Table 2.

| Tablet formulation | | Diameter (mm) (n=13) | Thickness (mm) (n=13) | Weight (mg) $\pm$ SD (n=13) | Hardness (N) $\pm$ SD (n=3) | Friability (%) | Porosity (%) |
|---|---|---|---|---|---|---|---|
| | FCC | 11.02 | 5.30 | 499.4 $\pm$ 2.9 | 117.3 $\pm$ 15.0 | 1.06 | 64 |
| I1 | FCC + 3% AcDiSol® | 11.03 | 5.41 | 498.9 $\pm$ 1.9 | 99.7 $\pm$ 9.6 | 1.32 | 64 |
| I2 | FCC + 3% Viva Star® | 11.01 | 5.36 | 502.3 $\pm$ 0.8 | 107.0 $\pm$ 3.5 | 1.67 | 63 |
| I3 | FCC + 3% Kolfidon® CL | 11.02 | 5.14 | 499.2 $\pm$ 1.0 | 116.7 $\pm$ 19.2 | 1.18 | 62 |
| I4 | FCC + 3% UICEL | 11.03 | 5.20 | 500.8 $\pm$ 1.6 | 111.7 $\pm$ 21.1 | 1.10 | 63 |
| | Barcroft™ CS90 | 11.07 | 5.47 | 851.7 $\pm$ 1.4 | 95.3 $\pm$ 1.2 | 1.12 | 35 |
| C1 | Barcroft™ CS90 + 3% AcDiSol® | 11.08 | 5.58 | 845.3$\pm$1.2 | 88.3$\pm$2.5 | 1.25 | 36 |

(continued)

| Tablet formulation | | Diameter (mm) (n=13) | Thickness (mm) (n=13) | Weight (mg) ± SD (n=13) | Hardness (N) ± SD (n=3) | Friability (%) | Porosity (%) |
|---|---|---|---|---|---|---|---|
| C2 | Bareroft™ CS90 + 3% Viva Star® | 11.08 | 5.50 | 845.9 ± 1.3 | 94.7 ± 2.9 | 1.14 | 35 |
| C3 | Bareroft™ CS90 + 3% Kollidon® CL | 11.07 | 5.51 | 833.0 ± 1.5 | 98.3 ± 3.8 | 1.12 | 36 |
| C4 | Bareroft™ CS90 + 3% UICEL | 11.06 | 5.49 | 836.9 ± 1.1 | 95.7 ± 0.6 | 1.20 | 36 |
| | FlowLac® | 11.05 | 5.32 | 594.4 ± 2.7 | 88.3 ± 3.2 | 1.46 | 24 |
| C5 | FlowLac® + 3% AcDiSol® | 11.06 | 5.33 | 593.3 ± 1.3 | 86.3±2.1 | 1.28 | 24 |
| C6 | FlowLac® + 3% Viva Star® | 11.06 | 5.34 | 598.7 ± 0.8 | 88.3 ± 5.0 | 1.40 | 23 |
| C7 | FlowLac® + 3% Kollidon® CL | 11.06 | 5.35 | 595.0 ± 1.2 | 89.3 ± 3.2 | 1.50 | 24 |
| C8 | FlowLac® + 3% UICEL | 11.06 | 5.34 | 605.4 ± 2.7 | 97.3 ± 7.8 | 1.16 | 23 |
| | MCC 102 | 11.06 | 5.53 | 489.0 ± 3.6 | 93.0 ± 7.5 | 0.54 | 40 |
| C9 | MCC 102 + 3% AcDiSol® | 11.06 | 5.55 | 494.0 ± 2.8 | 93.3 ± 2.1 | 0.38 | 40 |
| C10 | MCC 102 + 3% Viva Star® | 11.07 | 5.54 | 498.3 ± 2.3 | 96.0 ± 1.0 | 0.51 | 39 |
| C11 | MCC 102 + 3% Kollidon® CL | 11.06 | 5.55 | 487.9 ± 1.8 | 98.3±3.5 | 0.48 | 40 |
| C12 | MCC 102 + 3 % UICEL | 11.07 | 5.54 | 493.6 ±2.0 | 92.3 ± 3.5 | 0.59 | 40 |
| | UICEL | 11.10 | 5.83 | 566.6 ± 2.9 | 3.8 | 1.52 | 33 |
| C13 | UICEL + 3 % AcDiSol® | 11.09 | 5.75 | 563.3±1.9 | 94.0 ± 3.5 | 1.38 | 33 |
| C14 | UICEL + 3 % Viva Star® | 11.08 | 5.84 | 573.8 t 1.8 | 90.0 | 1.64 | 32 |
| C15 | UICEL + 3% Kollidon® CL | 11.10 | 5.82 | 566.5 ± 1.3 | 86.7 ± 3.8 | 1.53 | 33 |

Barcroft ™ CS 90, (SPI Pharma, Germany), PharMagnesia CC Type Natur 120, (Lehman & Voss & Co., Germany) are directly compressible natural calcium carbonate. FlowLac®100, (Meggle, Germany) is a lactose monohydrate. MCC 102 (is equivalent to MCC SANAQ ® 102 as previously described).

Table 2 presents the properties of the tablets. Concerning the weight with respect to the same volume and hardness (100 N), the tablets with FCC and MCC were the lightest (around 500 mg). By comparison, the CS90 tablets were around 1.7 times heavier (around 840 mg) than the tablets consisting of FCC and MCC. Friability was ca. 1-1.7% for all the tablets except the tablet formulations with MCC, where a friability of ca. 0.5% could be reached. Although the volume and hardness of the tablets were kept constant, the porosity of the tablets varied strongly between the different tablet formulations. The inventive tablet formulations I1-14 with FCC had a porosity of over 60% whereas the comparative MCC-based tablets C9-C12 could reach only 40% porosity at the same weight. With a porosity of about 25% and 35%, the comparative tablets C5-C8 FlowLac and C13-C15 UICEL were less porous than the MCC tablets. With a weight of around 840 mg, comparative formulations C1-C4 with CS90 showed a porosity of around 35%.

With calcium carbonate Natur 120, which is a natural ground calcium carbonate, no tablets could be produced with the desired properties. The hardness of 100 N was not reached due to capping of the tablets.

*Residence time and kinetic of water absorption (tensiometer)*

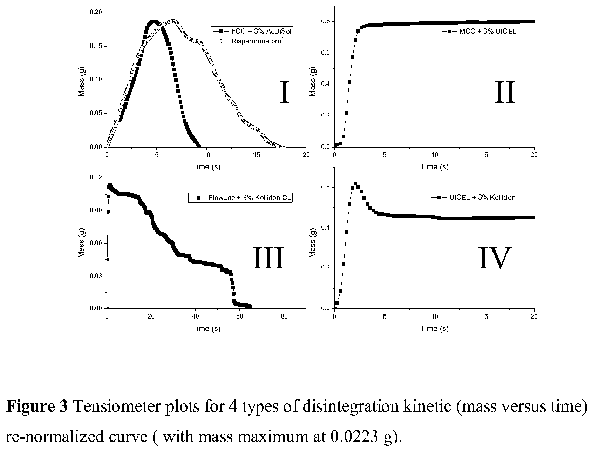

Tensiometer plots were categorized into four representative types of disintegration. Disintegration type I showed a profile, where in a first step the absorption of water outweighed the disintegration (increase in mass). After the peak was reached, the tablet continuously dispersed (decrease in mass) into very small particles. The following formulations belonged to disintegration type I: FCC + AcDiSol, FCC + VivaStar, FCC + Kollidon CL, MCC + VivaStar, UICEL + AcDiSol, UICEL + VivaStar, and Risperidone oro. The profile of disintegration type II was characterized by fast initial water absorption. After the saturation of the pores with water, the speed of the water absorption was more and more reduced. Some formulations reached a plateau, whereas other formulations were still able to absorb more water, forming a large swollen lump. Typical for this type of disintegration was that no disintegration occurred. Disintegration type II was observed in the following formulations: FCC + UICEL, FCC without disintegrants, CS90 + AcDiSol, CS90 + Kollidon CL, CS90 + UICEL, CS90 without disintegrants, MCC + UICEL, and MCC without disintegrants. The fastest water absorption in the initial phase was detected for type III disintegration profiles. After the peak, the water absorption passed into disintegration. In comparison to type I, the disintegration phase in type III was characterized by nonuniformity caused by larger parts that were falling off of the tablet and further through the mesh. These parts needed some more time on the bottom of the beaker to disperse completely. For this type of disintegration we could not exclude that the insides of the parts, which were fallen down, were still dry. The formulations given below showed a disintegration type III: CS90 + VivaStar, FlowLac + AcDiSol, FlowLac + VivaStar, FlowLac + Kollidon CL, FlowLac + UICEL, and FlowLac without disintegrants. Disintegration type IV was similar to type II. The initial phase was characterized by fast water absorption, followed by a peak. The major difference between type II and type IV was an initial disintegration phase after the peak. This disintegration phase was followed by level off the curve. Similar to disintegration type II, a complete disintegration was not possible. The following formulations belonged to disintegration type IV: MCC + AcDiSol, MCC + Kollidon CL, UICEL + Kollidon CL, and UICEL without disintegrants. Figure 3 shows a selection of the tensiometer plots for residence time. Table 3 shows the residence times and disintegration degrees obtained after the double linear curve fit from Figure 3. In addition, Table 3 presents the speed of water absorption and the amount of absorbed water after 90 s for comparison. Some formulations with FCC, MCC and UICEL were able to reach a water absorption speed of more than 50 mg/s. Only MCC and UICEL formulations absorbed water with a speed of more than 100 mg/s.

With respect to the same volume and hardness (100N), the formulations with UICEL showed the highest absolute amount of absorbed water.

Table 3. Calculated parameters for residence time, disintegration degree, and kinetic of water absorption

| Tablet formulation | Residence time (s) | Disintegration degree (%) | Disintegration type (I-IV) | Amount of absorbed water after 90 s (g) | Speed of water absorption (mg/s) |
|---|---|---|---|---|---|
| FCC | ∞ | 0 | II | 0.189 ± 0.011 | 4.5 ± 0.33 |
| FCC + 3% AcDiSol® | 8.92 | 100.0 | I | 1.232 ± 0.018 | 80.4 ± 2.69 |
| FCC + 3% Viva Star® | 11.94 | 100.0 | I | 1.599 ± 0.055 | 86.8 ± 3.95 |
| FCC + 3% Kollidon® CL | 9.53 | 100.0 | I | 0.816 ± 0.007 | 37.9 ± 0.92 |
| FCC + 3% UICEL | 4858.26 | 2.0 | II | 0.229 ± 0.008 | 4.9 ± 0.54 |
| Barcroft™ CS90 | ∞ | 0 | II | 0.115 ± 0.013 | 0.7 0.09 |
| Barcroft™ CS90 + 3% AcDiSol® | 7703.4 | 4.7 | II | 0.080 ± 0.033 | 1.9 ± 0.05 |

(continued)

| Tablet formulation | Residence time (s) | Disintegration degree (%) | Disintegration type (I-IV) | Amount of absorbed water after 90 s (g) | Speed of water absorption (mg/s) |
|---|---|---|---|---|---|
| Barcroft™ CS90 + 3% Viva Star® | 197.68 | 100.0 | III | 0.263 ± 0.015 | 5.9 ± 0.29 |
| Barcroft™ CS90 + 3% Kollidon® CL | ∞ | 0 | II | 0.074 ± 0.037 | 1.6 ± 0.24 |
| Barcroft™ CS90 + 3% UICEL | ∞ | 0 | II | 0.113 ± 0.021 | 1.1 ± 0.19 |
| FlowLac® | 61.92 | 100.0 | III | 0.311 ± 0.044 | 5.4 ± 2.06 |
| FlowLac® + 3% AcDiSol® | 127.85 | 100.0 | III | 0.322 ± 0.016 | 5.5 ± 0.29 |
| FlowLac® + 3% Viva Star® | 194.2 | 100.0 | III | 0.667 ± 0.026 | 16.2 ± 1.64 |
| FlowLac® + 3% Kollidon® CL | 65.09 | 100.0 | III | 0.375 ± 0.017 | 9.8 ± 0.33 |
| FlowLac® + 3% UICEL | 64.57 | 85.0 | III | 0.344 ± 0.045 | 9.1 ± 0.95 |
| MCC 102 | ∞ | 0 | II | 0.807 ± 0.040 | 79.2 ± 17.95 |
| MCC 102 + 3% AcDiSol® | 1681.78 | 47.2 | IV | 1.306 ± 0.017 | 82.3 ± 13.61 |
| MCC 102 + 3% Viva Star® | 9.65 | 99.1 | IV | 1.840 ± 0.050 | 152.9 ± 27.09 |
| MCC 102 + 3% Kollidon® CL | ∞ | 0 | IV | 0.877 ± 0.016 | 70.1 ± 15.83 |
| MCC 102 + 3% UICEL | ∞ | 0 | II | 0.847 ± 0.044 | 71.0 ± 12.97 |
| UICEL | ∞ | 0 | IV | 1.741 ± 0.059 | 96.6 ± 5.63 |
| UICEL + 3% AcDiSol® | 5.92 | 96.3 | IV | 1.864 ± 0.052 | 70.9 ± 3.22 |
| UICEL + 3% Viva Star® | 10.4 | 100.0 | I | 2.347 ± 0.034 | 98.1 ± 4.64 |
| UICEL + 3% Kollidon® CL | ∞ | 0 | IV | 1.826 ± 0.054 | 104.9 ± 13.24 |
| Risperidone oro | 17.26 | 100.0 | I | - | - |

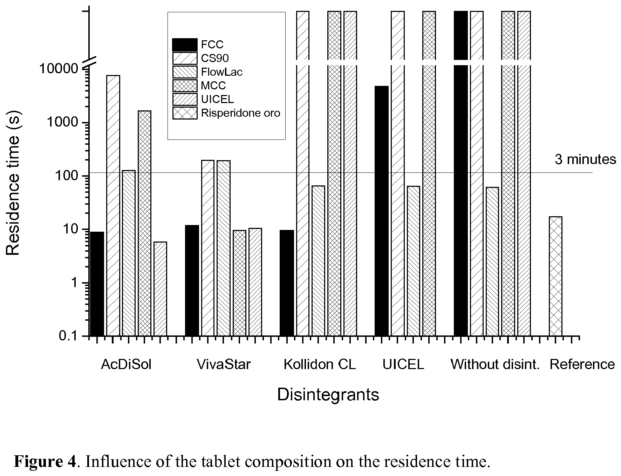

As mentioned before, an ODT should disintegrate within 3 minutes, if tested with the standard disintegration test according to the European Pharmacopeia. Figure 4 illustrates the influence of the tablet composition on the residence time. The horizontal line indicates a residence time of 3 minutes. With the binders FCC and FlowLac, three formulations in each case had a residence time of less than 3 minutes. In comparison with FlowLac, the FCC formulations had a significantly shorter residence time. Figure 4 presents that the FCC formulations showed fast disintegrating behavior which was comparable to UICEL and MCC formulations. It is important to note that the FCC formulations were well comparable to the reference risperidone oro tablets. On the other hand, it has to be kept in mind that FCC had to be used in combination with disintegrants to trigger the fast dispersing behavior. For all the tablets with a residence time below 3 minutes, a disintegration degree between 85% and 100% was calculated. Nevertheless, the results in Table 3 show that not all of the tablet formulations had residence times below 3 minutes. We marked the residence time values as "∞", if the values for the calculated residence time (Δt) indicated that the water absorption for the whole measuring period was not followed by disintegration stage.

Measurement Methods

*True Density*

The true density of FCC was determined by helium pycnometry (Micromeritics AccuPyc 1330, USA).

Tablet hardness

Tablet hardness was determined by measuring the crushing strength of a sample at homogeneous conditions in accordance with European and US Pharmacopeia with Tablet Tester 8M (Pharmatron, Switzerland).

*Tablet friability*

Friability of uncoated tablets was determined by measuring the weight of tablets before and after stress in the friability apparatus. The weight loss was calculated in percents. The experimental setup and operating conditions were in accordance with European and US Pharmacopeia. The friability apparatus Erweka (type TA200, Germany) was used.

*Pone size distribution*

The pore size distribution of FCC was determined with a mercury porosimeter (AutoPore IV 9500, Micromeritics Instrument, USA). Circa one third of the stem volume was filled with the sample. The low pressure mercury intrusion was in a pressure range from 3.59 kPa to 206.64 kPa. During the high pressure mercury intrusion, the pressure ranged from 206.64 kPa to 206.78 MPa. For both, the high-and low-pressure intrusion, an equilibration time of 10 seconds was adjusted.

*BET Specific Surface Area*

To measure the specific surface area, a Nova 2000e (Quantachrome Instruments, USA) was used with the five point BET method, a method well known to the skilled person. After degassing the samples for 12 hours at room temperature, the samples were measured with nitrogen at constant temperature (77.4 K). The measurement was performed in duplicate. BET (Brunaer, Emmet, Teller) method of measuring the specific surface area (e.g. in $g/m^2$) is based on the monolayer molecular gas adsorption (Langmuir theory). By obtaining the weight of gas monolayer the total covered surface is calculated. The standard multipoint (e.g. 5-point) BET procedure takes a minimum of 3 points in the measurement range. The BET equation is fitted with the obtained data points. The weight of the monolayer of adsorbate can be obtained from slope and intercept of resulting BET plot. [Source: NOVA Operation Manual, v8.0.]

*Particle size distribution*

Particle size distribution was determined with a Mastersizer X long bed (Malvern Instruments, UK). For MCC, UICEL, FlowLac, Barcroft, AcDiSol and VivaStar, the dry powder feeder (Malvern) was used. Kollidon and FCC were dispersed in isopropyl myristate and then analyzed (separately) by using the small volume sample presentation unit (Malvern). The samples were measured in triplicate, except for Kollidon which was measured in duplicate. The medians of the particle diameter and their standard deviations are shown.

The FCC samples were measured with the presentation 2_NFE. Such settings are readily derivable form the manual of the Malvern Mastersizer X. The number 2 as first character refers to the model X, N as second character refers to relative particle refractive index (real) of 1.095, F as third character refers to relative refractive index (imaginary) of 0.01, and E as fourth character refers to the dispersant refractive index of 1.5. With this presentation, the refractive index of Calciumcarbonate (ca. 1.6) and Isopropylmyristate (ca. 1.4) were taken into account. For the analysis "monomodal" setting was chosen.

For Kollidon the presentation 2NFE was chosen with the analysis "polydisperse". All the other measurements were carried out with the dry powder feeder. For these measurements the presentation 2RAA with a "polydisperse" analysis was chosen, wherein R= 1.45, A=0, A=1 as selected according to the manufacturers manual.

*Tablet characterization*

To determine the mean tablet weight, tablets (n=13) were weighted with an electronic balance (Mettler Toledo, type XS204 DeltaRange, Switzerland). The tablet diameter of 13 tablets was measured with a micrometer screw (Mitutoyo Model CD-15CPX, Japan) and the tablet thickness (n=13) was measured with a dial indicator (Compac type 532G, Switzerland).

Friability was measured by ERWEKA (type TA200, Germany) as above-decsribed.

The hardness of the tablets (n=3) was checked with a hardness tester (Tablet tester 8M, Pharmatron, Switzerland). To determine the true densities the helium pycnometer was used (Micromeritics AccuPyc 1330, USA). The porosity $\varepsilon$ (%) of the tablets was calculated with the Equation (1):

$$\varepsilon = \left(1 - \frac{\frac{m}{\rho}}{\pi \cdot r^2 \cdot h}\right) \cdot 100 \qquad (1)$$

where *m* is the tablet weight (g), p the true density of the powder mixture (g/cm$^3$), r the radius of the tablet (cm), and h the height of the tablet (cm).

*Kinetic of water absorption (tensiometer)*

The water absorption capacity of the tablets (n=3) for each lot was measured with a tensiometer (Krüss Processor Tensiometer K100MK2, Germany) in a water bath (37°C $\pm$ 1°C). The tablet was placed in a glass tablet holder with a ceramic filter bottom. With the help of the software, the time was plotted against the mass gain. The slope of this function lead to the speed of water absorption and the saturation level corresponded to the relative amount of absorbed water. To calculate the slope, the values for the time points between 6 and 9 seconds were taken into an account. OriginPro version 8.5 was used to evaluate the profiles.

*Method for characterization of disintegration and dispersion kinetics*

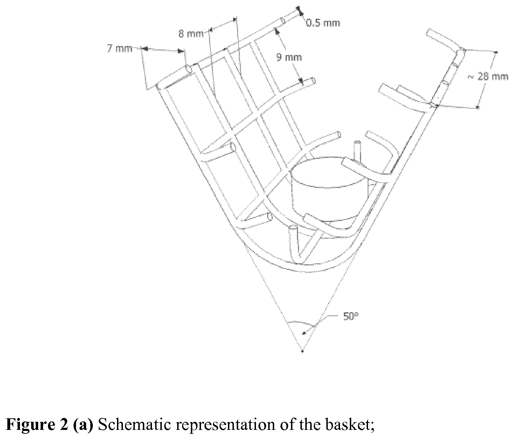

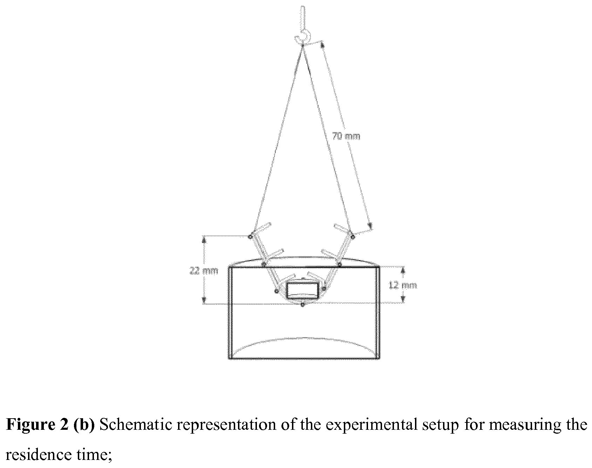

To characterize the disintegration and dispersion kinetics of the tablets (n=3, for FCC without disintegrants n=2) a tensiometer (Krüss Processor Tensiometer K100MK2, Germany) was used. The experimental setup was composed of a special metal-wire basket (Figure 2 (a)) which was attached to the microbalance of the tensiometer with four nickel wires. For the measurement of small tablets (as risperidone oro tablets), the mesh size was reduced by a nickel wire to size of 4 mm x 4.5 mm. As shown in Figure 2 (b), which is a schematic representation of the experimental setup for measuring the residence time, the basket was immersed to a defined depth (12 mm) into a beaker. The beaker was filled up to the edge with distilled water. The beaker was heated (37°C $\pm$ 1°C) by the surrounding thermostatic water bath.

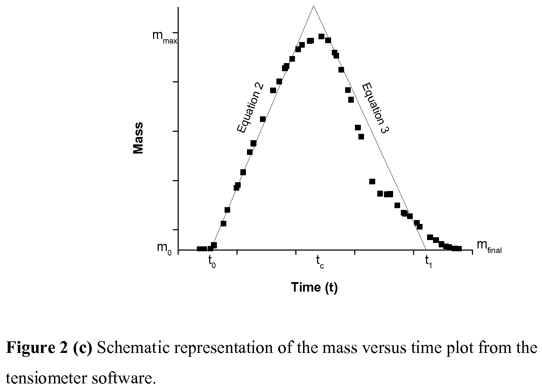

For the measurement, the weight loss versus time was recorded by the tensiometer software. A schematic representation of this plot is shown in Figure 2 (c), which is a schematic representation of the mass versus time plot from the tensiometer software. The tablet was placed manually on the basket immersed in the water. With the aid of the tensiometer software, the mass was plotted against the time. The time, when the tablet reached the basket and the disintegration together with water absorption started, was referenced as to. At this stage the weight was increased due to prevalence of water uptake. This was reflected as weight increase on the profiles. The weight decrease was explained as prevalence of disintegration upon water uptake. The leveling off of the profile was indicating the end of the disintegration. This event was referenced as $t_1$. The difference between $t_1$ and to ($t_1$-$t_0$) was referenced as tablet residence time on the basket. The reference time is a measure of disintegration time and is a good indicator of the time needed to disperse the tablet in the mouth cavity or a spoon. To determine the to and $t_1$, the fitting of the two linear equations was carried out with OriginPro version 8.5. A user defined double linear curve fit was programmed with the Equation (2).

$$m_{\text{absorption}} = m_0 + k_0 \cdot t \quad t < t_c \ (2)$$

$$m_{\text{elimination}} = m_0 + k_0 \cdot t_c + k_1(t - t_c) \quad t \geq t_c \ (2)$$

where *m* is the weight (g) and *t* is the time (s).

If $m_g$ and $m_g$ are set equal to 0 and the Equation 2 is solved for *t*, the following Equations are obtained:

$$t_0 = \frac{-m_0}{k_0}$$

$$t_1 = t_c - \frac{m_0 + k_0 \cdot t_c}{k_1}$$

To calculate the residence time, Equation 3 was used.

$$\Delta t = t_1 - t_0 = t_c - \frac{m_0 + k_0 \cdot t_c}{k_1} - \frac{-m_0}{k_0} \quad (3)$$

In addition to the residence time, the disintegration degree was calculated with Equation 4:

$$n = \left(1 - \frac{m_{final}}{m_{max}}\right) \cdot 100 \quad (4)$$

where n is the disintegration degree (%) and m is the weight (g). For $m_{max}$ the weight at the point $t_c$ was used and $m_{final}$ is the weight at leveling off of the profile (Figure 2 (c)).

**Claims**

1. A fast disintegrating dosage form in the form of a tablet comprising functionalized natural and/or synthetic calcium carbonate, at least one active ingredient and at least one disintegrant, wherein said functionalized natural or synthetic calcium carbonate is a reaction product of natural or synthetic calcium carbonate with carbon dioxide and one or more acids, wherein the carbon dioxide is formed in situ by the acid treatment and/or is supplied from an external source, wherein the at least one disintegrant is selected from the group comprising modified cellulose gums, insoluble cross-linked polyvinylpyrrolidones, starch glycolates or mixtures thereof, and wherein the tablet has a hardness in the range from 40 to 100 N and a corresponding tensile strength in the range of 0.4 to 1.3 MPa and disintegrates in less than or in 3 minutes, preferably in less than or in 2 minutes, more preferably in less than or in 1 minute, still more preferably in less than or in 30 seconds, when introduced into an aqueous environment.

2. A fast disintegrating dosage form according to claim 1, wherein the source of natural calcium carbonate is selected from the group of marble, calcite, chalk, limestone and dolomite and/or mixtures thereof.

3. A fast disintegrating dosage form according to claim 1, wherein the synthetic calcium carbonate is precipitated calcium carbonate (PCC) comprising aragonitic, vateritic or calcitic mineralogical crystals forms, especially prismatic, rhombohedral or scalenohedral PCC or mixtures thereof.

4. A fast disintegrating dosage form according to any of claims 1 to 3, wherein the acids are selected from the group of hydrochloric acid, sulfuric acid, sulfurous acid, hydrosulfate, phosphoric acid, phosphoric acid in combination with acetic, formic or citric acid or acid salts thereof, and mixtures thereof, preferably is phosphoric acid.

5. A fast disintegrating dosage form according to any of the claims 1 to 4, wherein the functionalized natural or synthetic calcium carbonate has BET specific surface area of from $5m^2/g$ to $200m^2/g$, preferably from $15m^2/g$ to $150m^2/g$, more preferably from $40m^2/g$ to $100m^2/g$, measured using nitrogen and the BET method according to ISO 9277:2010.

6. A fast disintegrating dosage form according to any of claims 1 to 5, wherein the functionalized natural or synthetic calcium carbonate has a weight median grain diameter $d_{50}$ of from 0.1 to 50 $\mu$m, preferably from 0.5 $\mu$m to 25 $\mu$m, more preferably from 0.8 $\mu$m to 20 $\mu$m, still more preferably from 1 $\mu$m to 15 $\mu$m measured using Malvern Mastersizer X long bed.

7. A fast disintegrating dosage form according to any of claims 1 to 6, wherein the functionalized natural or synthetic calcium carbonate has an intra-particle porosity determined as the pore volume per unit particle volume within the range of from 20 vol.% to 99 vol.%, preferably form 30 vol.% to 80 vol.%, more preferably from 40 vol.% to 70 vol.%, .-%, most preferably from 50 vol.% to 65 vol.%, calculated from mercury porosimetry measurement.

8. A fast disintegrating dosage form according to any of claims 1 to 7, wherein the at least one active ingredient is selected from the group comprising pharmaceutically active ingredients, inactive pharmaceutical precursors, biologically active ingredients, inactive biological precursors and/or mixtures thereof.

9. A fast disintegrating dosage form according to any of claims 1 to 8, further comprising natural or synthetic scenting agents, natural or synthetic flavoring agents, natural or synthetic coloring agents, natural or synthetic sweeteners and/or mixtures thereof.

10. A fast disintegrating dosage form according to any of claims 1 to 9, wherein the at least one disintegrant is present in the range from about 0.3 wt% to about 10 wt%, preferably from about 0.5 wt% to about 8 wt%, more preferably from about 1 wt% to about 5 wt% based on the weight of functionalized natural or synthetic calcium carbonate.

11. A fast disintegrating dosage form according to any of claims 1 to 10, wherein the fast disintegrating dosage form further comprises an effervescing agent.

12. A fast disintegrating dosage forms according to claim 11, wherein the effervescing agent is selected from the group comprising acids, acid salts, or hydrogen carbonates.

13. A method for producing the tablet of claims 1 to 12 by direct compression.

14. A method for producing the tablet of claims 1 to 12 by wet granulation in high-shear fluidized bed and subsequent compaction.

15. Use of functionalized natural or synthetic calcium carbonate in a fast disintegrating dosage form according to any claims 1 to 12.

**Patentansprüche**

1. Sich schnell auflösende Dosierungsform in der Form einer Tablette, umfassend funktionalisiertes natürliches und/oder synthetisches Calciumcarbonat, wenigstens einen Wirkstoff und wenigstens ein Sprengmittel, wobei das funktionalisierte natürliche oder synthetische Calciumcarbonat ein Reaktionsprodukt ist von natürlichem oder synthetischem Calciumcarbonat mit Kohlenstoffdioxid und einer oder mehreren Säuren, wobei das Kohlenstoffdioxid durch die Säurebehandlung in situ gebildet und/oder aus einer externen Quelle zugeführt wird, wobei das wenigstens eine Sprengmittel ausgewählt ist aus der Gruppe umfassend modifizierte Cellulose-Gummis, unlösliche, vernetzte Polyvinylpyrrolidone, Stärkeglykolate, oder Gemische davon, und wobei die Tablette eine Härte im Bereich 40 bis 100 N und eine entsprechende Bruchfestigkeit im Bereich von 0,4 bis 1,3 MPa aufweist, und sich in weniger oder in 3 Minuten auflöst, bevorzugt in weniger oder in 2 Minuten, bevorzugter in weniger oder in 1 Minute, noch bevorzugter in weniger oder in 30 Sekunden, wenn sie in eine wässrige Umgebung eingebracht wird.

2. Sich schnell auflösende Dosierungsform nach Anspruch 1, bei der die Quelle an natürlichem Calciumcarbonat ausgewählt ist aus der Gruppe von Marmor, Calcit, Kreide, Kalkstein und Dolomit und/oder Gemischen davon.

3. Sich schnell auflösende Dosierungsform nach Anspruch 1, bei der das synthetische Calciumcarbonat gefälltes Calciumcarbonat (PCC) ist, das aragonitische, vateritische oder calcitische mineralogische Kristallformen umfasst, insbesondere prismatisches, rhomboedrisches oder scalenoedrisches PCC oder Gemische davon.

4. Sich schnell auflösende Dosierungsform nach einem der Ansprüche 1 bis 3, bei der die Säuren ausgewählt sind aus der Gruppe von Salzsäure, Schwefelsäure, schwefliger Säure, Hydrogensulfat, Phosphorsäure, Phosphorsäure in Kombination mit Essig-, Ameisen- oder Zitronensäure oder Säuresalzen davon, sowie Gemischen davon, Phosphorsäure ist bevorzugt.

5. Sich schnell auflösende Dosierungsform nach einem der Ansprüche 1 bis 4, bei der das funktionalisierte natürliche oder synthetische Calciumcarbonat eine BET spezifische Oberfläche von 5 $m^2$/g bis 200 $m^2$/g aufweist, bevorzugt von 15 $m^2$/g bis 150 $m^2$/g, bevorzugter von 40 $m^2$/g bis 100 $m^2$/g, gemessen durch Verwenden von Stickstoff und dem BET Verfahren gemäss ISO 9277:2010.

6. Sich schnell auflösende Dosierungsform nach einem der Ansprüche 1 bis 5, bei der das funktionalisierte natürliche oder synthetische Calciumcarbonat einen gewichtsgemittelten Korndurchmesser $d_{50}$ von 0,1 bis 50 μm aufweist, bevorzugt von 0,5 bis 25 μm, bevorzugter von 0,8 bis 20 μm, noch bevorzugter von 1 bis 15 μm, gemessen durch Verwenden von Malvern Masersizer X Langbett.

7. Sich schnell auflösende Dosierungsform nach einem der Ansprüche 1 bis 6, bei der das funktionalisierte natürliche oder synthetische Calciumcarbonat eine Intrapartikelporosität, bestimmt als das Porenvolumen pro Einheitpartikelvolumen, im Bereich von 20 Vol.-% bis 99 Vol.-% aufweist, bevorzugt von 30 Vol.-% bis 80 Vol.-%, bevorzugter von 40 Vol.-% bis 70 Vol.-%, am meisten bevorzugt von 50 Vol.-% bis 65 Vol.-%, berechnet aus Quecksilberporositätsmessungen.

8. Sich schnell auflösende Dosierungsform nach einem der Ansprüche 1 bis 7, bei der der wenigstens eine Wirkstoff ausgewählt ist aus der Gruppe umfassend pharmazeutische Wirkstoffe, inaktive pharmazeutische Vorstufen, biologische Wirkstoffe, inaktive biologische Vorstufen und/oder Gemische davon.

9. Sich schnell auflösende Dosierungsform nach einem der Ansprüche 1 bis 8, ferner umfassend natürliche oder synthetische Duftstoffe, natürliche oder synthetische Aromastoffe, natürliche oder synthetische Farbstoffe, natürliche oder synthetische Süssungsmittel und/oder Gemische davon.

10. Sich schnell auflösende Dosierungsform nach einem der Ansprüche 1 bis 9, bei der das wenigstens eine Sprengmittel im Bereich von etwa 0,3 Gew.-% bis etwa 10 Gew.-% vorhanden ist, bevorzugt von etwa 0,5 Gew.-% bis etwa 8 Gew.-%, bevorzugter von etwa 1 Gew.-% bis etwa 5 Gew.-%, bezogen auf das Gewicht des funktionalisierten natürlichen oder synthetischen Calciumcarbonats.

11. Sich schnell auflösende Dosierungsform nach einem der Ansprüche 1 bis 10, bei der die sich schnell auflösende Dosierungsform ferner einen Sprudelstoff umfasst.

12. Sich schnell auflösende Dosierungsform nach Anspruch 11, bei der der Sprudelstoff ausgewählt ist aus der Gruppe umfassend Säuren, Säuresalze, oder Hydrogencarbonate.

13. Verfahren zur Herstellung der Tablette von den Ansprüchen 1 bis 12 durch Direktverpressen.

14. Verfahren zur Herstellung der Tablette von den Ansprüchen 1 bis 12 durch Nassgranulierung in einem hochscherenden Wirbelbett und nachfolgender Kompaktierung.

15. Verwendung von funktionalisiertem natürlichem oder synthetischem Calciumcarbonat in einer sich schnell auflösenden Dosierungsform gemäss einem der Ansprüche 1 bis 12.

**Revendications**

1. Forme posologique se désagrégeant rapidement sous la forme d'un comprimé comprenant du carbonate de calcium fonctionnalisé naturel et/ou synthétique, au moins un principe actif et au moins un agent de désagrégation, dans laquelle ledit carbonate de calcium fonctionnalisé naturel ou synthétique est un produit réactionnel de carbonate de calcium naturel ou synthétique avec du dioxyde de carbone et un ou plusieurs acides, dans laquelle le dioxyde de carbone est formé in situ par traitement acide et/ou est alimenté à partir d'une source externe, dans laquelle l'agent de désagrégation est choisi dans le groupe comprenant les gommes de cellulose modifiées, les polyvinylpyrrolidones réticulées insolubles, les glycolates d'amidon ou des mélanges de ceux-ci, et dans laquelle le comprimé a une dureté comprise dans la plage allant de 40 à 100 N et une résistance à la traction correspondante comprise dans la plage allant de 0,4 à 1,3 MPa et se désagrège en moins de ou en 3 minutes, de préférence en moins de ou en 2 minutes, plus préférablement en moins de ou en 1 minute, encore plus préférablement en moins de ou en 30 secondes, lorsqu'il est introduit dans un environnement aqueux.

2. Forme posologique se désagrégeant rapidement selon la revendication 1, dans laquelle la source de carbonate de calcium naturel est choisie dans le groupe constitué par le marbre, la calcite, la craie, le calcaire et la dolomite et/ou des mélanges de ceux-ci.

3. Forme posologique se désagrégeant rapidement selon la revendication 1, dans laquelle le carbonate de calcium synthétique est du carbonate de calcium précipité (PCC) comprenant les formes cristallines minéralogiques aragonitiques, vatéritiques ou calcitiques, en particulier du PCC prismatique, rhomboédrique ou scalénoédrique ou des mélanges de ceux-ci.

4. Forme posologique se désagrégeant rapidement selon l'une quelconque des revendications 1 à 3, dans laquelle les acides sont choisis dans le groupe constitué par l'acide chlorhydrique, l'acide sulfurique, l'acide sulfureux, l'hydrosulfate, l'acide phosphorique en combinaison avec de l'acide acétique, formique ou citrique ou des sels d'acides de ceux-ci, et des mélanges de ceux-ci, de préférence l'acide phosphorique.

5. Forme posologique se désagrégeant rapidement selon l'une quelconque des revendications 1 à 4, dans laquelle le carbonate de calcium fonctionnalisé naturel ou synthétique a une surface spécifique BET de 5 m2/g à 200 m2/g, de préférence de 15 m2/g à 150 m2/g, plus préférablement de 40 m2/g à 100 m2/g, mesurée en utilisant de l'azote et la méthode BET selon la norme ISO 9277:2010.

6. Forme posologique se désagrégeant rapidement selon l'une quelconque des revendications 1 à 5, dans laquelle le carbonate de calcium fonctionnalisé naturel ou synthétique a un diamètre de grain médian en poids d50 de 0,1 à 50 μm, de préférence de 0,5 à 25 μm, plus préférablement de 0,8 à 20 μm, encore plus préférablement de 1 à 15 μm, mesuré en utilisant le lit long Malvern Mastersizer X.

7. Forme posologique se désagrégeant rapidement selon l'une quelconque des revendications 1 à 6, dans laquelle le carbonate de calcium fonctionnalisé naturel ou synthétique a une porosité intraparticulaire, déterminée comme le volume de pore par le volume de particule unitaire, comprise dans la plage allant de 20 % en volume à 99 % en volume, de préférence de 30 % en volume à 80 % en volume, plus préférablement de 40 % en volume à 70 % en volume, de manière préférée entre toutes de 50 % en volume à 65 % en volume, calculée à partir d'une mesure de porosité au mercure.

8. Forme posologique se désagrégeant rapidement selon l'une quelconque des revendications 1 à 7, dans laquelle le principe est choisi dans le groupe comprenant les principes pharmaceutiquement actifs, les précurseurs pharmaceutiques inactifs, les principes biologiquement actifs, les précurseurs biologiques inactifs et/ou des mélanges de ceux-ci.

9. Forme posologique se désagrégeant rapidement selon l'une quelconque des revendications 1 à 8, comprenant en outre des agents parfumants naturels ou synthétiques, des agents aromatisants naturels ou synthétiques, des agents colorants naturels ou synthétiques, des édulcorants naturels ou synthétiques et/ou des mélanges de ceux-ci.

10. Forme posologique se désagrégeant rapidement selon l'une quelconque des revendications 1 à 9, dans laquelle l'agent de désagrégation est présent à hauteur d'environ 0,3 % en poids à environ 10 % en poids, de préférence d'environ 0,5 % en poids à 8 % en poids, plus préférablement d'environ 1 % en poids à 5 % en poids, par rapport au poids du carbonate de calcium fonctionnalisé naturel ou synthétique.

11. Forme posologique se désagrégeant rapidement selon l'une quelconque des revendications 1 à 10, dans laquelle la forme posologique se désagrégeant rapidement comprend en outre un agent effervescent.

12. Forme posologique se désagrégeant rapidement selon la revendication 11, dans laquelle l'agent effervescent est choisi dans le groupe comprenant les acides, les sels d'acides ou les hydrogénocarbonates.

13. Procédé de production du comprimé selon l'une quelconque des revendications 1 à 12 par compression directe.

14. Procédé de production du comprimé selon l'une quelconque des revendications 1 à 12 par granulation humide dans un lit fluidisé à fort cisaillement et compactage ultérieur.

15. Utilisation de carbonate de calcium fonctionnalisé naturel ou synthétique dans une forme posologique se désagrégeant rapidement selon l'une quelconque des revendications 1 à 12.

**Figure 1 (a)** SEM picture of FCC of the present invention with scale bar of 50 μm.

**Figure 1 (b)** SEM picture of FCC of the present invention with scale bar of 5 µm.

**Figure 1 (c)** SEM picture of FCC of the present invention with scale bar of 1 μm.

**Figure 1 (d)** Illustrative mercury porosimetry plot of FCC of the present invention.

**Figure 2 (a)** Schematic representation of the basket;

**Figure 2 (b)** Schematic representation of the experimental setup for measuring the residence time;

**Figure 2 (c)** Schematic representation of the mass versus time plot from the tensiometer software.

**Figure 3** Tensiometer plots for 4 types of disintegration kinetic (mass versus time) re-normalized curve ( with mass maximum at 0.0223 g).

**Figure 4**. Influence of the tablet composition on the residence time.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 4134943 A
- US 5595761 A
- US 5635210 A
- US 5807576 A
- US 6066337 A
- WO 2010037753 A
- WO 0039222 A
- US 20040020410 A1
- EP 2264108 A
- EP 2070991 B1
- WO 2004083316 A

### Non-patent literature cited in the description

- **C.J.RIDGWAY et al.** Modified calcium carbonate coatings with rapid absorption and extensive liquid uptake capacity. *Colloids and Surfaces A: Physicochemical and Engineering Aspects,* April 2004, vol. 236 (1-3), 91-102
- *Transport in Porous Media,* 2006, vol. 63, 239-259